# NEW EUROPEAN PATENT SPECIFICATION

(11) **EP 2 026 854 B2**
(45) Date of publication and mention of the opposition decision: **13.07.2016**
(45) Mention of the grant of the patent: 18.08.2010
(21) Application number: 07794680.4
(22) Date of filing: 08.05.2007
(51) Int. Cl.: A61L 31/02, A61L 31/14

(54) **DEGRADABLE MEDICAL DEVICE**
ABBAUBARE MEDIZINISCHE VORRICHTUNG
DISPOSITIF MÉDICAL DÉGRADABLE

(30) Priority: 22.05.2006 US 438925
(43) Date of publication of application: 25.02.2009
(73) Proprietor: Abbott Cardiovascular Systems Inc., Santa Clara, CA 95054-2807 (US)
(72) Inventor: KLEINE, Klaus, Los Gatos, CA 95033 (US); KRAMER-BROWN, Pamela, San Jose, CA 95135 (US)
(74) Representative: Silcock, Peter James
(86) International application number: PCT/US2007/011177
(87) International publication number: WO 2007/139668

(56) References cited:
- US-A1- 2002 004 060
- US-A1- 2002 082 679
- US-A1- 2004 088 038
- US-A1- 2005 209 680
- US-A1- 2006 229 711
- US-B1- 6 287 332

## Description

### BACKGROUND OF THE INVENTION

The present invention relates generally to stents which are adapted for implantation into a patient's body lumen and which are intended to degrade after implantation to eventually become absorbed and/or eliminated by the body. More particularly, the invention is applicable to a stent for deployment in a blood vessel in which its presence is only temporarily required.

Various medical devices are routinely implanted in a body lumen such as a blood vessel, wherein a permanent presence is not required and wherein an extended presence may actually be counterproductive. For example, stents are particularly useful in the treatment and repair of blood vessels after a stenosis has been compressed by percutaneous transluminal coronary angioplasty (PTCA), percutaneous transluminal angioplasty (PTA), or removed by atherectomy or other means, to help improve the results of the procedure and maintain patency. Alternatively, stents can be used to provide primary compression to a stenosis in cases in which no initial PTCA or PTA procedure is performed. It has however been found that the support that is provided by a stent is only required for a limited period of time, perhaps on the order of months, as the part of the vessel affected by stenosis would thereafter typically remain open even without any further support. The continued presence of some scent structures would then only serve as a permanent irritation of the tissue surrounding the stent, as the stent's rigidity could preclude it from performing the flexions caused by the heartbeat. An additional complication arises in pediatric applications because the stent comprises a fixed obstruction at the implantation site while such implantation site evolves with the growth of the child. Invasive retrieval of a stent is generally not considered to be a viable option.

While stents have typically been constructed of relatively inert metals in order to ensure their longevity, degradable stent structures have more recently been devised in an effort to provide support for only a limited period of time. Various polymeric substances are known that gradually dissolve and are absorbed by the body without adverse effect which has prompted the construction of stents with such polymers and polymer combinations for the purpose of providing only temporary support. It is however difficult to match the structural and mechanical properties of a metallic structure with the use of polymers, especially when polymeric materials are loaded with a drug, as drug loading of a polymeric material can have a significant adverse effect on strength. The need to minimize delivery profile as well as the desire to minimize bulk upon deployment substantially precludes simply increasing the dimensions of a polymeric stent in an effort to match the strength of a metallic structure.

It has more recently been found that certain metals, such as iron, are readily absorbable by the body without adverse effect. Consequently, the use of corrodible metals is being considered for use in degradable stent applications. Unfortunately, the corrosion rates of heretofore considered metallic structures have not been sufficiently high so as to provide for as limited a residence time as may be desirable in certain applications. Simply reducing the dimensions of a metallic implantable medical device in order to reduce residence times may not be a viable option due the compromise in strength that necessarily results. An approach is therefore needed for accelerating the corrosion rate of a metallic structure without unacceptably compromising strength in order to limit the residence time of such device within the body. Moreover, it is most desirable to control the degradation of the device such that full strength is retained for a preselected period of time after which corrosion proceeds at an accelerated rate.

US2005/209680 discloses implantable medical devices, such as stents, that include a metallic region composed of a bioerodible metal and a polymer region composed of a biodegradable polymer contacting the metallic region. Examples of biodegradable metals include Mg, Zn and Fe. The metallic region may be a pure metal or a mixture that includes two types of metals and if may comprise pores.

US2002/082679 discloses medical devices such as stents which are formed of metals or alloys degradable in the body with small pits or pin holes to accelerate corrosion. The stent may be coated with a polymeric material and a therapeutic agent.

US2004/088038 discloses porous metal stents for controlled release of therapeutic drugs, formed from a material selected from the group consisting of stainless steel, titanium, tantalum, nickel-titanium, cobalt-chromium, and alloys thereof and having a polymeric coating.

US2006/229711 discloses degradable medical devices comprising an implantable metal-body having at least one surface, and at least one corrosion inducing feature (such as pores) on the surface which causes at least a portion of the body to degrade at a rate greater than the rate without the feature in a physiologic environment.

WO 03/063733 discloses an expandable stent for insertion into a body passage having a mesh structure of interconnecting portions joined together by joining portions forming a galvaric couple.

### SUMMARY OF THE INVENTION

The present invention provides a degradable metallic stent which is configured to degrade at a sufficiently high rate so as to substantially limit its residence time within a body lumen in which it had been deployed. The stent is formed of porous metal, wherein the metal is selected for its propensity to corrode upon contact with the bodily fluids in which it is immersed without adversely affecting the body, while the porosity is relied upon to increase the surface area in contact with such fluids and thereby accelerate the rate of its corrosion. By selecting the metal and the degree of porosity, rates of degradation can be tailored to a wide range of applications.

The metal selected for use in the construction of a stent in accordance with the present invention comprises a combination of metals. Generally, the metal(s) must be implantable without causing significant inflammation, neointimal proliferation or thrombotic events and must be corrodible so as to dissolve, dissociate or otherwise break down in the body without ill effect. "Degradable", "biodegradable", "biologically degradable", "erodable", "bioabsorbable" and "bioresorbable" are all terms that have been used to describe this essential property.

The corrosion rate of a relatively slowly corroding metal can be accelerated by combining it with another metal selected so as to provide for a relatively high internal galvanic couple to yield a correspondingly high galvanic corrosion rate.

Reliance on galvanic corrosion in order to achieve a desired corrosion rate requires the selection of a metal pair that has a sufficiently high rest potential differential. A rest potential differential results from two metals that, by themselves, each have a particular rest potential when measured versus a reference electrode, for example a Standard Calomel Electrode (SCE) or Natural Hydrogen Electrode (NHE), in the same type of solution, for example saline or equine (horse) serum. The driving force toward corrosion that results from this differential may be tailored to control the rate of degradation of the joined materials. For example, a driving force of about 500 mV would generally result in a slower dissolution than a driving force of 1 V or more. Appropriate metal pairs can be selected from among the elements Mg, Mn, K, Ca, Na, Zn, Cr, Fe, Cd, Al, Co, Sb, V, Cu and Mo, and from alloys based on such elements.

The degree of porosity that is imparted to the combination of metals selected for use in the construction of the stent is an essential element for the practice of the present invention. The porosity has a substantial effect on the rate of corrosion to the extent that the ratio of corrosion rate increase to surface area increase has been found to vary from 0.3 to 1.0 depending on the type of material and the environment to which it is exposed. The morphology of the microcellular porous metal, including the cell size and porosity of the metal, can be controlled so that the cell sizes can be made very uniform, and can be controlled precisely by the manipulation of various parameters during the formation process. The desired porosity is achievable by a variety of techniques including, but not limited to sintering, foaming, extrusion, thixomolding, semi-solid slurry casting and thermal spraying. The stent structure may be formed using any of the well known techniques, including, for example, the laser cutting of a tubular form.

The corrosion of the porous metallic stent can additionally be modified with the application of a polymeric coating thereto. A coating with a degradable polymer serves to delay and/or reduce the corrosion of the underlying metal structure. For a fully degradable stent, utilizing a degradable polymer, the performance of a coated device can be tailored so as to maintain up to its full structural strength for an initial period of time followed by more rapid degradation thereafter. The corrosion rates of selected portions of a stent can additionally be differentiated with the application of either degradable and/or non-degradable polymeric coatings to only portions of the stent.

The present invention additionally provides for the controlled release of therapeutic drugs by a degradable metallic stent with the loading of such drugs directly into t pore structure of the stent, or alternatively, with the loading of drug-loaded polymers onto or into the porous stent.

Other features and advantages of the present invention will become more apparent from the following detailed description when taken in conjunction with the accompanying exemplary drawings.

### BRIEF DESCRIPTION OF THE DRAWINGS

FIGURE 1 is an elevational view, partially in section, of a stent embodying features of the invention which is mounted on a delivery catheter and disposed within a damaged artery.
FIG. 2 is an elevational view, partially in section, similar to that shown in FIG. 1 wherein the stent is expanded within a damaged artery.
FIG. 3 is an elevational view, partially in section, depicting the expanded stent within the artery after withdrawal of the delivery catheter.

### DETAILED DESCRIPTION OF PREFERRED EMBODIMENTS

FIG.1 generally depicts a corrodible metal stent 10, incorporating features of the invention, mounted on a catheter assembly 12 which is used to deliver the stent and implant it in a body lumen, such as a coronary artery, carotid artery, peripheral artery, or other vessel or lumen within the body. The stent generally comprises a plurality of radially expandable cylindrical rings 11 disposed generally coaxially and interconnected by undulating links 15 disposed between adjacent cylindrical elements. The catheter assembly includes a catheter shaft 13 which has a proximal end 14 and a distal end 16. The catheter assembly is configured to advance through the patient's vascular system by advancing over a guide wire by any of the well known methods of an over the wire system (not shown) or a well known rapid exchange catheter system, such as the one shown in FIG. 1.

Catheter assembly 12 as depicted in FIG. 1 is of the well known rapid exchange type which Includes an RX port 20 where the guide wire 18 will exit the catheter. The distal end of the guide wire 18 exits the catheter distal end 16 so that the catheter advances along the guide wire on a section of the catheter between the RX port 20 and the catheter distal end 16. As is known in the art, the guide wire lumen which receives the guide wire is sized for receiving various diameter guide wires to suit a particular application. The stent is mounted on the expandable member 22 (balloon) and is crimped tightly thereon so that the stent and expandable member present a low profile diameter for delivery through the arteries. Alternatively, the invention may be practiced using a self-expanding stent configuration as is well known in the art.

As shown in FIG. 1, a partial cross-section of an artery 24 is shown with a small amount of plaque that has been previously treated by an angioplasty or other repair procedure. Stent 10 of the present invention is used to repair a diseased or damaged arterial wall which may include the plaque 25 as shown in FIG. 1, or a dissection, or a flap which are commonly found in the coronary arteries, carotid arteries, peripheral arteries and other vessels.

In a typical procedure to implant stent 10, the guide wire 18 is advanced through the patient's vascular system by well known methods so that the distal end of the guide wire is advanced past the plaque or diseased area 25. Prior to implanting the stent, the cardiologist may wish to perform an angioplasty procedure or other procedure (e.g., atherectomy) in order to open the vessel and remodel the diseased area. Thereafter, the stent delivery catheter assembly 12 is advanced over the guide wire so that the stent is positioned in the target area. The expandable member or balloon 22 is inflated by well known means so that it expands radially outwardly and in turn expands the stent radially outwardly until the stent is apposed to the vessel wall. The expandable member is then deflated and the catheter withdrawn from the patient's vascular system. The guide wire typically is left in the lumen for post-dilatation procedures, if any, and subsequently is withdrawn from the patient's vascular system. As depicted in FIGS. 2 and 3, the balloon is fully inflated with the stent expanded and pressed against the vessel wall, and in FIG. 3, the implanted stent remains in the vessel after the balloon has been deflated and the catheter assembly and guide wire have been withdrawn from the patient.

The stent 10 serves to hold open the artery 24 after the catheter is withdrawn, as illustrated by FIG. 3. Due to the formation of the stent from an elongated tubular member, the undulating components of the stent are relatively flat in transverse cross-section so that when the stent is expanded, it is pressed into the wall of the artery and as a result does not interfere with the blood flow through the artery. The stent is pressed into the wall of the artery and will eventually be covered with endothelial cell growth which further minimizes blood flow interference. The undulating portion of the stent provides good tacking characteristics to prevent stent movement within the artery, Furthermore, the closely spaced cylindrical elements at regular intervals provide uniform support for the wall of the artery, and consequently are well adapted to tack up and hold in place small flaps or dissections in the wall of the artery, as illustrated in FIGS. 2 and 3.

The stent patterns shown in FIGS. 1-3 are for illustration purposes only and can vary in size and shape to accommodate different vessels or body lumens. Further, the metallic stent 10 is of a type that can be used in accordance with the present invention.

The stent illustrated in FIGS. 1-3 is formed of a corrodible metal and has a porous structure. The metal is selected for its propensity to corrode when subjected to bodily fluids and to break down in the body without ill effect.

The corrodible metal comprises a combination of two metals selected to create a galvanic couple such that the material will undergo galvanic dissolution upon contact with bodily fluids. The degradation rate may be tailored by selecting a combination of metals that have a driving force of about 500 mV or greater. In a most preferred embodiment the driving force would be about 1 V or greater. For example, Ti has a rest potential of 3.5 V vs. SCE in equine serum, and would, when paired with almost any other metal, yield a suitable driving force. Alternatively, the pairings Nb-Cr (1.1 V rest potential differential vs. SCE in equine serum), Pd-W (1.23 V rest potential vs. SCE in equine serum) and Cr-W (630 mV rest potential differential vs. SCE in equine serum) would also yield suitable driving forces.

Any of a variety of well-known manufacturing techniques can be relied upon to achieve a sufficient degree of porosity in the metallic structure be it a single element such as iron or a Nb-Cr pairing. Such techniques include but are not limited to sintering, extrusion, thixomolding, semi-solid casting and thermal spraying. A preferred method comprises the formation of microcellular metallic foams as developed at Massachusetts Institute of Technology and Clarkson University, as out-lined in V. Kumar and N. P. Sub, Polym. Eng. Sci., 30, pp. 1323-1329 (1990), and C. Wang, K. Cox and G. Campbell, J. Vinyl Additives Tech., 2(2), pp. 167-169 (1996). Such microcellular foams are typically characterized by cell sizes or diameters in the range of 0.1 to 100 microns, and cell densities in the range of 109 to 1015 cells per cubic cm. The foaming process can be carried out on metallic preforms such as extruded hypotubing of a desired dimension. The first stage of microcellularfoam processing involves dissolving an inert gas, such as nitrogen or CO2, under pressure into the metallic matrix. The next phase is the rapid creation of microvoids. This is initiated by inducing large thermodynamic instability by quickly decreasing the solubility of the gas in the metal by changing the pressure or temperature. Other various techniques known in the art can be used to fabricate microcellular porous metal. For example, microcellular porous metal can be fabricated by employing the technique of powder technology which involves mixing a select polymer with metal powder and using an injection molding process to shape the tube or the stent preform. Alternatively, an electrolytic process for the deposition of a metal onto a polymerfoam precursor by way of electrolytic deposition can be used to fabricate porous metal. The morphology of the microcellular porous metal, including the cell size and porosity of the metal, can be controlled so that the cell sizes can be made very uniform, and can be controlled precisely by changing thermodynamic variables like pressure and temperature during formation of the microcellular porous metal. The microcellular porous metal can be formed by a batch process that can be easily controlled and operated, in which extruded tubing can be cut to the desired lengths and then foamed in a separate pressure chamber.

After a tube of porous metal has been formed, a stent as illustrated in the Figures is manufactured by for example laser cutting the tube so as to remove material and leave portions of the metallic tubing which are to form the rings, struts and links. In accordance with the invention, it is preferred to cut the tubing in the desired pattern using a machine-controlled laser which process is well known in the art. After laser cutting, the stent rings are subjected to a surface smoothing mechanism such as bead blasting with a safe media, honing, etc. Electropolishing is also an option, although the solution used must be selected so as to minimize degradation, an example of which is ELECTRO-GLO #300, sold by the ELECTRO-GLO Co., Inc. in Chicago, Illinois, which is a mixture of sulfuric acid, carboxylic acids, phosphates, corrosion inhibitors and a biodegradable surface active agent. The bath temperature, current density and cathode to anode area are selected according to principles well known in the art.

A bioabsorbable polymer coating may additionally be applied about the exterior of the porous structure in order to delay the corrosion process of the underlying metallic structure. Suitable polymers include but are not limited to polyalkanoates (PHA), poly(3-hydroxyal-kanoates), such as poly(3-hydroxypropanoate), poly(3-hydroxybutyrate) (PHB), poly(3-hydroxyvalerate) (PHV), poly(3-hydroxybutyrate-ω-3-hydroxyvalerate) (PHBV), poly(3-hydroxyhexanoate), poly(3-hydroxyheppanoate) and poly(3-hydroxyoctanoate), poly(4-hydroxyalkanoate) such as poly(4-hydroxybutyrate), poly(4-hydroxyvalerate), poly(4-hydroxyhexanoate), poly(4-hydroxyheptanoate), poly(4-hydroxyoctanoate) and copolymers comprising any of the 3-hydroxyalkanoate or 4-hydroxyalkanoate monomers described herein or blends thereof, polyesters, poly(DL-lactide), poly(L-lactide), polyglycolide, poly(lactide-co-glycolide), polycaprolactone, poly(lactide-co-caprolactone), poly(glydolide-co-caprolactone), poly(dioxanone), poly(ortho esters), poly(anhydrides), poly(tyrosine carbonates) and derivatives thereof, poly(tyrosine ester) and derivatives thereof, poly(imino carbonates), poly(phosphoesters), poly(phosphazenes), poly(amino acids), polysaccharides, collagen, chitosan, alginate, and PolyAspirin.

Stents relying on galvanic corrosion to achieve an accelerated degradation rate in accordance with the present invention are preferably formed of element or alloy combinations with at least about 500 mV of driving force, more preferably with at least about 800 mV of driving force and most preferably with at least about 1 V of driving force. The porosity of the metal structure of such stents is at least 60%.

## Claims

1. An implantable, biodegradable medical device having a porous, corrodible metal structure, wherein said medical device is a stent,
wherein said metal structure has a porosity of at least 60%, and
wherein said metal structure comprises a combination of two metals that form one or more internal galvanic couples.

2. The implantable medical device of claim 1, wherein said two metals form an internal couple with a driving force of at least about 500 mV.

3. The implantable medical device of claim 1, wherein a degradable polymeric coating is applied to at least a portion of the medical device.

4. The implantable medical device of claim 3, wherein said degradable polymeric coating is applied to the entire medical device.

5. The implantable medical device of claim 3, wherein said polymeric coating is drug loaded.

## Patentansprüche

1. Implantierbare, biologisch abbaubare medizinische Vorrichtung mit einer porösen, korrodierbaren Metallstruktur, wobei die medizinische Vorrichtung ein Stent ist,
wobei die besagte Metallstruktur eine Porosität von mindestens 60% aufweist, und
wobei die besagte Metallstruktur eine Kombination aus zwei Metallen enthält, die eine oder mehrere interne galvanische Kopplungen bilden.

2. Implantierbare medizinische Vorrichtung gemäß Anspruch 1, bei der die besagten zwei Metalle eine interne galvanische Kopplung mit einer treibenden Kraft von mindestens zirka 500 mV bilden.

3. Implantierbare medizinische Vorrichtung gemäß Anspruch 1, bei der zumindest auf einem Teil der medizinischen Vorrichtung eine abbaubare Polymerbeschichtung aufgebracht wird.

4. Implantierbare medizinische Vorrichtung gemäß Anspruch 3, bei der die besagte abbaubare Polymerbeschichtung auf der ganzen medizinischen Vorrichtung aufgebracht wird.

5. Implantierbare medizinische Vorrichtung gemäß Anspruch 3, bei der die besagte Polymerbeschichtung Arzneimittelträger ist.

## Revendications

1. Dispositif médical biodégradable implantable ayant une structure poreuse corrosive en métal, où ledit dispositif médical est un stent,
où ladite structure en métal a une porosité d'au moins 60 %, et
où ladite structure en métal comprend une combinaison de deux métaux qui forment un ou plusieurs couples galvaniques internes.

2. Dispositif médical implantable selon la revendication 1, dans lequel lesdits deux métaux forment un couple interne avec une force motrice d'au moins 500 mV environ.

3. Dispositif médical implantable selon la revendication 1, dans lequel un revêtement polymère dégradable est appliqué sur au moins une partie du dispositif médical.

4. Dispositif médical implantable selon la revendication 3, dans lequel ledit revêtement polymère dégradable est appliqué sur la totalité du dispositif médical.

5. Dispositif médical implantable selon la revendication 3, dans lequel ledit revêtement polymère est chargé d'un médicament.
